# EUROPÄISCHE PATENTSCHRIFT

(11) **EP 0 565 847 B1**
(45) Veröffentlichungstag und Bekanntmachung des Hinweises auf die Patenterteilung: **02.07.1997**
(21) Anmeldenummer: 93103533.1
(22) Anmeldetag: 05.03.1993
(51) Int. Cl.: A61C 5/02, A61C 5/00

(54) **Vorrichtung für eine Wurzelspitzenresektion**
Device for root resection
Dispositif pour la résection apicale

(30) Priorität: 19.03.1992 DE 9203684 U
(43) Veröffentlichungstag der Anmeldung: 20.10.1993
(73) Patentinhaber: Pohl, Yango, 35390 Giessen (DE)
(72) Erfinder: Pohl, Yango, 35390 Giessen (DE)
(74) Vertreter: Missling, Arne, Dipl.-Ing.

(56) Entgegenhaltungen:
- DE-A- 3 124 981
- GB-A- 220 491
- GB-A- 2 178 319
- US-A- 2 174 796
- US-A- 4 060 081
- US-A- 4 906 670
- DEUTSCHE ZAHN RZTLICHE ZEITSCHRIFT, Band 29, Nr. 9, September 1974, MUnchen G. WILSTERMANN "Der retro- grade Wurzelkanalverschluss mit Goldent" Seiten 759-760

## Beschreibung

Die Erfindung betrifft ein Mittel zur stofflichen Trennung der Oberfläche einer während einer Wurzelspitzenresektion freigelegten Knochenkavität gemäß dem Oberbegriff von Anspruch 1.

Der Ablauf einer solchen Wurzelspitzenresektion ist ausführlich beschrieben bei Kirschner, Atlas der chirurgischen Zahnerhaltung, München/Wien 1987, Seite 130 bis Seite 135. Dabei wird nach Abheben der Knochenschleimhaut die Umgebung der zu resezierenden Wurzelspitze soweit von Knochensubstanz befreit, wie das zur Ausführung der Operation erforderlich ist. Die entstehende Knochenkavität hat eine der Wurzelspitze entsprechende charakteristische Raumform. Nachdem ungefähr 20% der Gesamtwurzellänge reseziert worden sind, wird zumeist eine retrograde Wurzelkanalkavität hergestellt, die ebenso wie der Wurzelkanal selbst zu verfüllen ist. Dabei kann in den Wurzelkanal ein Stift inseriert sein, so daß nur wenig Füllmaterial gebraucht wird.

Intraoperative Wurzelfüllungen erfordern die Spülung des Wurzelkanales mit hochreaktiven Reagenzien, wie 3%-igem H₂O₂, 5%igem NaOCl, Alkohol usw. Diese Spülungen treten an der Resektionsfläche aus und werden dort abgesaugt, wobei sich aber nicht vermeiden läßt, daß sie teilweise auch in die Knochenkavität gelangen. Auch bei der retrograden Füllung, die manchmal erforderlich ist, muß die Wurzelkavität mit hochreaktiven Reagenzien, z.B. 40%iger Zitronensäure, vorbereitet werden. Auch dabei läßt es sich oft nicht vermeiden, daß Flüssigkeit in die Knochenkavität gelangt. Andererseits sind die Füllungen in eine trockene Wurzelkavität bzw. einen vollständig trockenen Wurzelkanal einzubringen und müssen geraume Zeit ohne Zutritt von Flüssigkeit aushärten können. Da die Füllungen im Überschuß eingebracht werden müssen, läßt es sich wegen der Konsistenz dieser Füllungen auch oft nicht vermeiden, daß Teile davon in die Knochenkavität gelangen und mühsam daraus wieder entfernt werden müssen.

Aus der die Knochenkavität begrenzenden Oberfläche tritt Sickerblut aus, das die Trocknung sowohl der Wurzelkavität als auch des Wurzelkanales vor dem Einbringen der Füllungen verhindert und die Füllungsmaterialien in ihrer Aushärtephase kontaminiert.

Damit trotzdem eine einwandfreie Wurzelspitzenresektion durchführbar ist, muß man entweder die Wurzelkavität mit Gaze straff austamponieren, in die Knochenkavität Reagenzien applizieren, die durch vasokonstriktorische oder adstringierende Wirkung die Blutung stillen, oder die Knochenkavität mit Knochenwachs auskleiden. Dabei ist die Applikation von Adrenalin oder dergleichen wegen langer Einwirkzeit, unsicherem Wirkungseintritt, unsicherer Wirkungsdauer und gefährlicher Nebenwirkungen nicht vorteilhaft, zumal bei jedem zwischenzeitlichen Ausspülen der Knochenkavität erneute Blutung auftreten kann. Die Abdeckung der Knochenoberfläche mit Hilfe von Knochenwachs, die bei einer möglichst trockenen Knochenkavität durchgeführt werden muß, d.h. nach dem Applizieren blutstillender Reagenzien, verhindert zwar weiteren Blutaustritt, sie hat jedoch den Nachteil, daß die Wachspartikel nach der Operation wieder entfernt werden müssen, was in mühevoller Handarbeit durchgeführt werden muß.

Um diese Mängel abzustellen, ist es aus Wilstermann, Der retrograde Wurzelkanalverschluß mit Goldent, Deutsche Zahnärztliche Zeitschrift 29 (1974), Seiten 759 bis 760, bereits bekannt, über den freigelegten Wurzelquerschnitt die Ringöse eines mit einem Handgriff versehenen Ringinstrumentes zu legen, die den Resektionsbereich von der umgebenden retrograd präparierten Knochenkavität soweit trennt, daß die Wurzelkavität und der Wurzelkanal trocken bleiben. Dabei ist allerdings eine Sichteinschränkung für den Operateur unvermeidlich, und auch der Zugang zur Resektionsfläche ist eingeschränkt. Außerdem muß während der gesamten Behandlungszeit dieses Ringinstrument gehalten werden. Damit ist aber offensichtlich die Arbeit für den Operateur wesentlich erschwert.

Die Erfindung hat sich demzufolge die Aufgabe gestellt, ein Mittel der eingangs näher bezeichneten Art so zu gestalten, daß es während der gesamten Operation in situ verbleibt, ohne gehalten werden zu müssen, daß es freie Sicht und freien Zugang zur resezierten Fläche der Wurzelspitze und zur Wurzelkavität bzw. zum Wurzelkanal erlaubt, leicht und billig herstellbar und in die Knochenkavität einführbar ist und in einfacher Weise deren verschiedenen Raumformen angepaßt werden kann.

Erfindungsgemäß wird die Aufgabe durch den in Anspruch 1 definierten Gegenstand gelöst.

Weitere Merkmale der Erfindung enthalten die Unteransprüche.

Das erfindungsgemäße Mittel realisiert die umrissene Aufgabenstellung in überraschend einfacher Weise. Eine derartige Auskleidung kann allen vorkommenden Raumformen bequem angepaßt werden, entweder durch verschiedene Patrizen, durch eine elastische Zwischenschicht oder durch manuelle Formung außerhalb der Knochenkavität, insbesondere, wenn diese sehr groß ist, so daß die Verwendung einer Patrize unbequem ist. Die Patrize wird vorteilhaft dünnwandig ausgebildet, so daß der Wurzelkanal beim Einführen der Auskleidung in die Knochenkavität gut sichtbar ist. Es ist nunmehr eine vollständige stoffliche Trennung des umgebenden Knochenmaterials von der Zahnwurzel gewährleistet, wobei die Auskleidung in die Knochenkavität einlegbar ist, sobald diese ausgearbeitet ist, und ohne weiteres darin verbleiben kann, bis die Wurzelfüllung ausgehärtet ist und die Operation beendet wird. Sie behindert die erforderlichen Manipulationen nicht und stört auch nicht deren visuelle Beobachtung. Die Schichten können ohne weiteres so ausgeführt werden, daß keinerlei Partikel ausgerissen werden können und in der Knochenkavität verbleiben. Das zwischen der Auskleidung und dem Knochengewebe aus den Knochenkanälen austretende Blut, das dort auch gerinnt, trägt zur besseren Haftung der Auskleidung bei. Die Auskleidung selbst läßt sich, insbesondere als flachbahniges Rohteil, in großer Stückzahl sehr billig herstellen und vor Ort mittels Scheren oder dergleichen paßgerecht konfektionieren. Selbst im Bereich der an die Resektionsfläche anschließenden Knochenpartie wird ein Blutaustritt sicher verhindert, wenn die leicht plastische, blutstillende und/oder flüssigkeitsaufsaugende Schicht über die Ränder der für die Form verantwortlichen Schicht gezogen ist, so daß auch die Raumzwickel miterfaßt werden.

Die Erfindung wird nachfolgend an einem Ausführungsbeispiel anhand der Zeichnung näher erläutert. Es zeigen
- Fig. 1: eine Gesamtansicht einer durch eine künstlich geschaffene Knochenkavität freigelegten und resezierten Wurzelspitze einer Zahnwurzel, wobei ein erfindungsgemäßes Mittel in diesem Bereich eingelegt ist, in räumlicher Darstellung,
- Fig. 2: eine Draufsicht auf Fig. 1,
- Fig. 3: einen Schnitt I-I aus Fig. 2,
- Fig. 4: einen Schnitt II-II aus Fig. 2,
- Fig. 5: eine Ansicht wie Fig. 2, jedoch vor dem Einbringen des erfindungsgemäßen Mittels,
- Fig. 6: eine Ansicht wie Fig. 5, während des Einbringens des Mittels, und
- Fig. 7: eine besondere Ausgestaltung des Mittels nach der Erfindung,
sämtlich in schematischer, vereinfachter Darstellung.

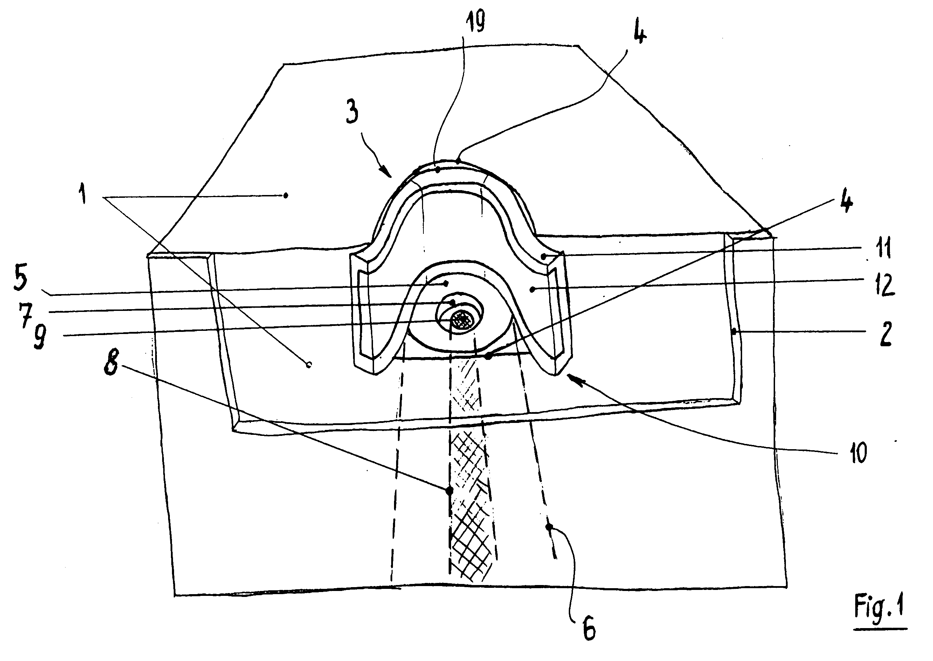

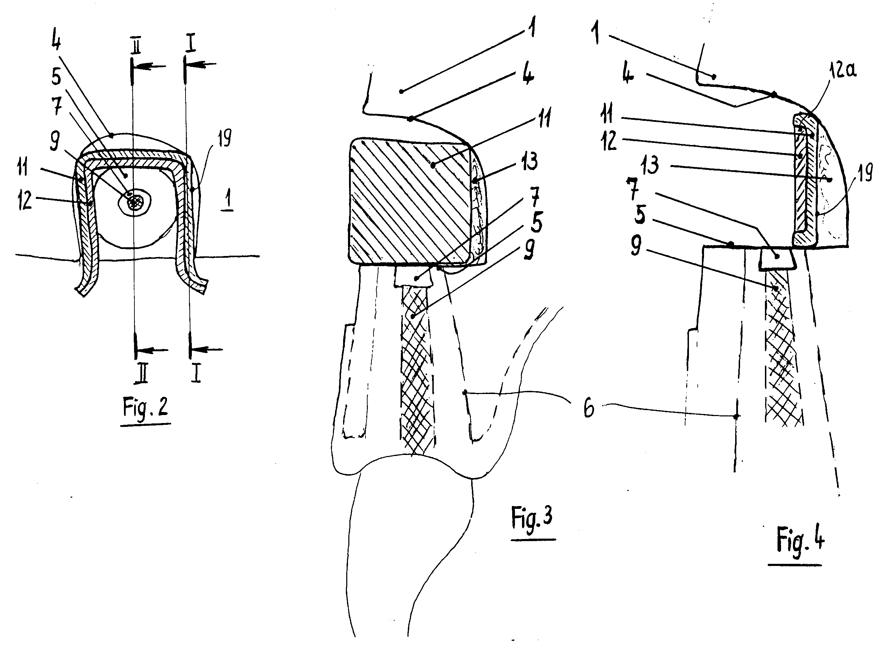

Fig. 1 zeigt die Partie des Kieferknochens im Bereich der resezierten Wurzelspitze. Dabei ist der Knochen 1 von dem anschließenden Mukoperiostlappen soweit freigeschnitten, daß der Operationsbereich freigelegt ist. Der abgehobene Lappen selbst ist weggelassen, jedoch ist die zugehörige Schnittstelle 2 zu erkennen. Aus dem Knochen 1 ist eine Knochenkavität 3 herausgearbeitet, von der in Fig. 1 ihre angeschnittene Oberfläche 4 zu erkennen ist. Die Form der Knochenkavität 3 ist insbesondere aus den Fig. 2 bis 4 zu erschließen. Die Wurzelspitzenresektion ist in Fig. 1 bereits durchgeführt, so daß eine Resektionsfläche 5 sichtbar ist. Eine Wurzelkavität 7, die in das Dentin der Zahnwurzel 6 eingearbeitet ist, ist für eine retrograde Füllung vorgesehen. Der Wurzelkanal 8 ist in der Situation der Fig. 1 bereits mit Wurzelfüllmaterial 9 verfüllt, was sowohl im sichtbaren Bereich der Wurzelkavität als auch im an sich unsichtbaren Wurzelkanal durch eine Kreuzschraffung symbolisiert ist. In der Knochenkavität 3 befindet sich eine erfindungsgemäße Auskleidung 10, die aus zwei Schichten 11 und 12 zusammengesetzt ist. In der Draufsicht der Fig. 2 und den in Fig. 3 und Fig. 4 gezeigten, um 90° gedrehten vergrößerten Schnitten I-I und II-II aus Fig. 2 sind die Einzelheiten der Auskleidung noch besser zu erkennen. In Fig. 4 ist dabei auch zu sehen, daß die knochenseitige Schicht 11 die zweite, kavitätsseitige Schicht 12 an deren Rändern 12a umfaßt; da erfindungsgemäß die Schicht 11 sehr leicht plastifizierbar ist, findet auf diese Weise eine einwandfreie Abdichtung der Knochenkavität 3 von der Oberfläche 4 des Knochens 1 statt, insbesondere in der Ebene der Resektionsfläche 5. Das an den Bereichen der Oberfläche 4, die nicht direkt von der Schicht 11 abgedeckt sind, austretende, aber gerinnende Blut 13 (Fig. 3 und 4) sorgt dafür, daß aus dem angeschnittenen Knochen 1 kein weiteres Blut austreten und den Fortgang der Operation stören kann.

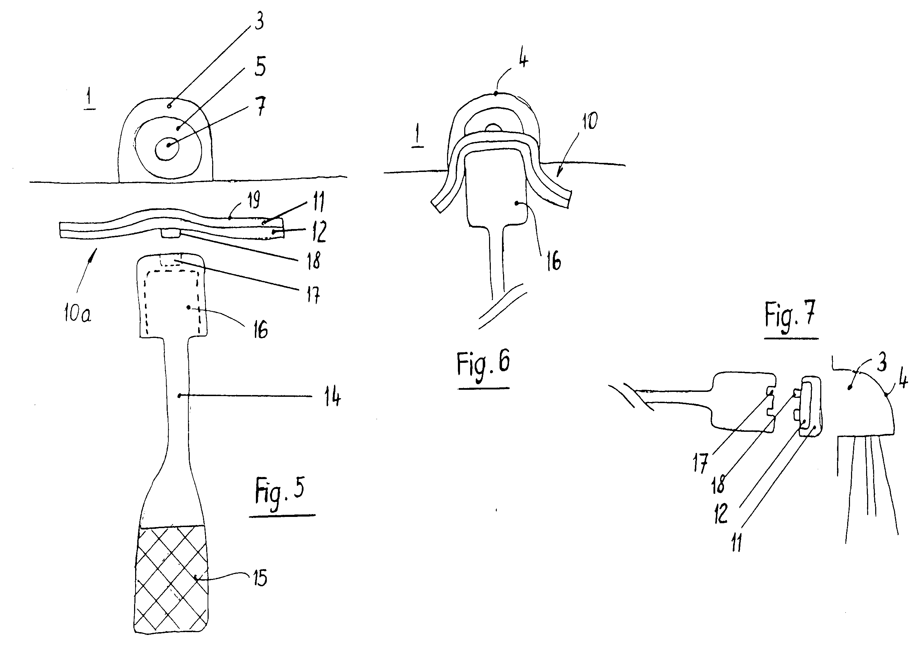

In den Fig. 5 und 6 ist stark vereinfacht dargestellt, wie das Rohteil 10a einer Auskleidung 10 mit Hilfe eines Stempels 14 manuell in die Knochenkavität 3 eindrückbar ist, wobei das Rohteil 10a zur endgültigen Form der Auskleidung 10 umgeformt wird. Der Stempel 14 weist außer einem Griff 15 noch eine Patrize 16 auf, die das eigentliche Umformwerkzeug für das Rohteil 10a bildet. Es ist in Fig. 5 zu sehen, daß die Patrize 16 durch Formschluß mit der Auskleidung 10 bzw. dessen Rohteil 10a in Verbindung kommt, hier durch eine Ausnehmung 17 in der Patrize 16 und einen zugehörigen Butzen 18 an der Auskleidung 10 auf der Seite der Schicht 12. Eine formschlüssige Verbindung zwischen der Auskleidung 10 bzw. deren Schicht 12 und der Patrize 16 ist in vielfältiger Weise möglich, wie ein weiteres Beispiel in Fig. 7 zeigt. Dort sind mehrere Paarungen von Ausnehmungen 17 und Butzen 18 vorgesehen. Stattdessen können auch Leisten oder ringförmige, formschlüssige Verbindungen verwendet werden.

Anstelle der in den Fig. 5 bis 7 dargestellten Verformung der Auskleidung 10 mit Hilfe eines Stempels 14 bzw. einer Patrize 16 ist es auch möglich, insbesondere dann, wenn die Knochenkavität 3 sehr groß ist und beispielsweise die Bereiche mehrerer Zahnwurzeln 6 umfaßt, eine solche Auskleidung 10 außerhalb des Mundes, der Knochenkavität in etwa angepaßt, von Hand auszuformen. Nach eventuell erforderlichen Korrekturen kann die Auskleidung 10 auch in dieser Form funktionsgerecht verwendet werden.

Die Schicht 11 soll sehr leicht plastisch verformbar, blutstillend und/oder flüssigkeitsaufsaugend sein, insbesondere auf ihrer der Oberfläche 4 der Knochenkavität 3 zugekehrten Seite 19. Sie besteht zweckmäßigerweise aus regenerierter, oxydierter Zellulose oder nativem, bovinem Kollagen oder Thrombin. Hingegen wird für die Schicht 12 ein passender metallischer Werkstoff oder Kunststoff verwendet, der gegebenenfalls aushärtbar gewählt wird. Wesentlich ist, daß diese Schicht 12 nach ihrer Verformung eine Restelastizität in einer Größe bewahrt, die ausreichend ist, daß die Auskleidung 10 in der Knochenkavität 3 mit wenn auch geringfügiger Vorspannung festgehalten wird, so daß sie nach ihrer Lokalisation vom Operateur "vergessen" werden kann. Die beiden Schichten 11 und 12 sind miteinander fest verbunden, beispielsweise durch Verklebung mit Fibrin.

Es ist auch möglich, daß zwischen den Schichten 11 und 12 eine Zwischenschicht vorgesehen ist, die aus einem elastisch-plastischen Werkstoff besteht und so dick ausgeführt ist, daß sich die relativ dünne Schicht 11 der Oberfläche 4 besser anpaßt und gegebenenfalls dort vorhandene Unebenheiten ausgleicht. Selbstverständlich müssen alle verwendeten Werkstoffe sterilisierbar sein. Es hat sich gezeigt, daß eine Dicke der Auskleidung 10 von 0,5 bis 1,0 mm für den Anwendungszweck optimal ist.

## Patentansprüche

1. Mittel zur stofflichen Trennung der Oberfläche einer während einer Wurzelspitzenresektion freigelegten Knochenkavität über der Wurzelspitze einer Zahnwurzel von der Resektionsfläche an der Zahnwurzel, dem freigelegten Wurzelkanal und einer an dessen Ende und zu seinem Verschluß auf der Seite der Knochenkavität ausgearbeiteten Wurzelkavität, insbesondere während einer Spülung und Verfüllung des Wurzelkanals und der Wurzelkavität, wobei
das Mittel als eine dünnwandige Auskleidung (10) ausgebildet ist, welche so dimensioniert ist, daß sie geeignet ist, die Oberfläche (4) der Knochenkavität (3) nahezu vollständig abzudecken, daß die Auskleidung (10) plastisch verformbar und mit geringem Kraftaufwand der räumlichen Form der Oberfläche (4) anpaßbar ist, dadurch gekennzeichnet, daß die Auskleidung (10) eine Restelastizität von einer Größenordnung besitzt, durch die sie nach ihrer Lokalisation in der Knochenkavität (3) mit einer geringen Vorspannung gehaltert wird, und daß sie auf ihrer der Oberfläche (4) der Knochenkavität (3) zugekehrten Seite (19) aus einem blutstillenden und/oder flüssigkeitsaufsaugenden Werkstoff ausgeführt ist.

2. Mittel nach Anspruch 1, dadurch gekennzeichnet, daß die Auskleidung (10) als flachbahniges Rohteil (10a) hergestellt ist und in der Knochenkavität (3) durch eine deren Raumform angepaßte Patrize (16) ausgeformt wird, die vorteilhaft dünnwandig ausgebildet ist.

3. Mittel nach Anspruch 1, dadurch gekennzeichnet, daß die Auskleidung (10) aus einem flachbahnigen Rohteil (10a) hergestellt und derart vorgeformt ist, daß sie etwa der Raumform der Knochenkavität (3) folgt.

4. Mittel nach einem der Ansprüche 1 bis 3, dadurch gekennzeichnet, daß die Auskleidung (10) mehrschichtig ausgeführt ist.

5. Mittel nach Anspruch 4, dadurch gekennzeichnet, daß die Auskleidung (10) aus zwei Schichten (11, 12) besteht, die miteinander vorzugsweise fest verbunden sind.

6. Mittel nach Anspruch 5, dadurch gekennzeichnet, daß eine knochenseitige Schicht (11) blutstillend und/oder flüssigkeitsaufsaugend und sehr leicht plastifizierbar und eine diese Schicht (11) tragende, kavitätsseitige Schicht (12) unter einer Verformungskraft ebenfalls plastifizierbar, aber eine gewisse Restelastizität behaltend ausgeführt ist.

7. Mittel nach einem der Ansprüche 1 bis 6, dadurch gekennzeichnet, daß die Auskleidung (10) für feste und flüssige Stoffe unpassierbar ausgeführt ist.

8. Mittel nach einem der Ansprüche 1 bis 7, dadurch gekennzeichnet, daß zwischen der knochenseitigen Schicht (11) und der kavitätsseitigen Schicht (12) zumindest in einem Teilbereich eine elastische oder plastische Zwischenschicht vorgesehen ist, die so dick ausgeführt ist, daß Unebenheiten der Oberfläche (4) der Knochenkavität (3) ausgleichbar sind.

9. Mittel nach einem der Ansprüche 1 bis 8, dadurch gekennzeichnet, daß die kavitätsseitige Schicht (12) und/oder die Zwischenschicht für feste und flüssige Stoffe unpassierbar ausgeführt sind.

10. Mittel nach einem der Ansprüche 1 bis 9, dadurch gekennzeichnet, daß die Dicke der Auskleidung (10) zwischen 0,5 und 1,0 mm beträgt.

11. Mittel nach einem der Ansprüche 1 bis 10, dadurch gekennzeichnet, daß die Auskleidung (10) aus einem sterilisierbaren Werkstoff besteht.

12. Mittel nach einem der Ansprüche 1 bis 11, dadurch gekennzeichnet, daß die knochenseitige Schicht (11) die Zwischenschicht und/oder die kavitätsseitige Schicht (12) zumindest in einem Teilbereich an deren Rändern (12a) einfaßt.

13. Mittel nach einem der Ansprüche 1 bis 12, dadurch gekennzeichnet, daß die knochenseitige Schicht (11) aus regenerierter, oxydierter Zellulose oder nativem, bovinem Kollagen oder Thrombin besteht.

14. Mittel nach einem der Ansprüche 1 bis 13, dadurch gekennzeichnet, daß die kavitätsseitige Schicht (12) aus Metall oder Kunststoff besteht.

15. Mittel nach einem der Ansprüche 1 bis 14, dadurch gekennzeichnet, daß die Patrize (16) durch Formschluß mit der Auskleidung (10) verbindbar ist.

## Claims

1. Means for the material separation of the surface of a bone cavity - exposed during a radiectomy and located above the root apex of a dental root - from the resection surface at the dental root, from the exposed root canal and from a root cavity which is to occlude the root canal at the side nearest the bone cavity and is made at the end of the root canal, in particular during rinsing and filling-in of the root canal and the root cavity, the means being in the form of a thin-walled lining (10) dimensioned in such a manner that it is suitable to cover virtually the entire surface (4) of the bone cavity (3), the lining (10) being plastically deformable and being adaptable to the three-dimensional form of the surface (4) with a low expenditure of force, characterised in that the lining (10) has a residual resilience of an order of magnitude through which after its localisation it is fixed in position in the bone cavity (3) with slight prestressing, and in that on its side (19) facing the surface (4) of the bone cavity (3) it is made of a blood-staunching and/or fluid-absorbing material.

2. Means in accordance with Claim 1, characterised in that the lining (10) is manufactured as a flat-sheet unmachined part (10a) and is shaped in the bone cavity (3) by a punch (16) which is adapted to the three-dimensional form thereof and is advantageously thin-walled.

3. Means in accordance with Claim 1, characterised in that the lining (10) is manufactured from a flat-sheet unmachined part (10a) and is preformed in such a manner that it approximately follows the three-dimensional form of the bone cavity (3).

4. Means in accordance with any one of Claims 1 to 3, characterised in that the lining (10) is multi-layered.

5. Means in accordance with Claim 4, characterised in that the lining (10) is composed of two layers (11, 12) which are preferably securely connected to one another.

6. Means in accordance with Claim 5, characterised in that a layer (11) located at the side nearest the bone is blood-staunching and/or fluid-absorbing and very easily plasticizable, and a layer (12) which is located at the side nearest the cavity and bears this layer (11) is - under a deformation force - likewise plasticizable, retaining however a certain residual resilience.

7. Means in accordance with any one of Claims 1 to 6, characterised in that solid and fluid substances cannot pass through the lining (10).

8. Means in accordance with any one of Claims 1 to 7, characterised in that at least in a partial region, a resilient or plastic intermediate layer is provided between the layer (11) located at the side nearest the bone and the layer (12) located at the side nearest the cavity, this intermediate layer being such in thickness that unevenness of the surface (4) of the bone cavity (3) can be evened out.

9. Means in accordance with any one of Claims 1 to 8, characterised in that solid and fluid substances cannot pass through the layer (12) located at the side nearest the cavity and/or the intermediate layer.

10. Means in accordance with any one of Claims 1 to 9, characterised in that the lining (10) is between 0.5 and 1.0 mm thick.

11. Means in accordance with any one of Claims 1 to 10, characterised in that the lining (10) is composed of a sterilizable material.

12. Means in accordance with any one of Claims 1 to 11, characterised in that the intermediate layer and/or the layer (12) located at the side nearest the cavity are enclosed at least in a partial region - at their edges (12a) - by the layer (11) located at the side nearest the bone.

13. Means in accordance with any one of Claims 1 to 12, characterised in that the layer (11) located at the side nearest the bone is composed of regenerated, oxidised cellulose or native, bovine collagen or thrombin.

14. Means in accordance with any one of Claims 1 to 13, characterised in that the layer (12) located at the side nearest the cavity is composed of metal or a plastics material.

15. Means in accordance with any one of Claims 1 to 14, characterised in that the punch (16) is connectable to the lining (10) by a positive connection.

## Revendications

1. Moyen de séparation matérielle de la paroi d'une cavité osseuse, située au-dessus de la pointe d'une racine de dent et ouverte pendant une résection de pointe de racine, de la surface de résection sur la racine de dent, du canal ouvert de la racine et d'une cavité de racine préparée à son extrémité et jusqu'à son raccordement sur le côté de la cavité osseuse, en particulier pendant un lavage et un remplissage du canal de racine et de la cavité de racine,
ce moyen étant réalisé sous la forme d'un revêtement (10) à paroi mince, qui est dimensionné de telle façon qu'il soit susceptible de recouvrir presque complètement la paroi (4) de la cavité osseuse (3), le revêtement (10) étant déformable plastiquement et ajustable, en appliquant une force légère, à la forme prise dans l'espace par la paroi (4),
caractérisé en ce que
le revêtement (10) présente une élasticité résiduelle d'un ordre de grandeur maintenant ce revêtement, après sa mise en place, avec une faible précontrainte, dans la cavité osseuse (3),
et en ce que ce revêtement est réalisé, sur sa face (19) tournée vers la paroi (4) de la cavité osseuse (3), avec une matière coagulant le sang et/ou aspirant les liquides.

2. Moyen suivant la revendication 1, caractérisé en ce que le revêtement (10) est réalisé sous la forme d'un élément brut en bande plate (10a), et est mis en forme dans la cavité osseuse (3) au moyen d'une spatule (16) adaptée à la forme du volume de cette cavité, cette spatule étant avantageusement réalisée avec une faible épaisseur.

3. Moyen suivant la revendication 1, caractérisé en ce que le revêtement (10) est réalisé sous la forme d'un élément brut en bande plate (10a), et est préformé de façon à suivre sensiblement la forme du volume de la cavité osseuse (3).

4. Moyen suivant l'une des revendications 1 à 3, caractérisé en ce que le revêtement (10) est constitué de plusieurs couches.

5. Moyen suivant la revendication 4, caractérisé en ce que le revêtement (10) est constitué de deux couches (11, 12), qui sont avantageusement reliées solidairement l'une à l'autre.

6. Moyen suivant la revendication 5, caractérisé en ce qu'une couche (11) située du côté de l'os est conçue pour coaguler le sang et/ou pour aspirer les liquides, et est très facilement plastifiable, et en ce qu'une couche (12), située du côté de la cavité et portant cette couche (11), est, de même, réalisée plastifiable sous une force de formage, tout en conservant une certaine élasticité résiduelle.

7. Moyen suivant l'une des revendications 1 à 6, caractérisé en ce que le revêtement (10) est réalisé de façon à ne pas laisser passer les matières solides ou liquides.

8. Moyen suivant l'une des revendications 1 à 7, caractérisé en ce qu'entre la couche (11) située du côté de l'os et la couche (12) située du côté de la cavité, il est prévu, au moins dans une zone partielle, une couche intermédiaire, élastique ou plastique, réalisée assez épaisse pour compenser des irrégularités de la paroi (4) de la cavité osseuse (3).

9. Moyen suivant l'une des revendications 1 à 8, caractérisé en ce que la couche (12) située du côté de la cavité, et/ou la couche intermédiaire, sont réalisées de façon à ne pas laisser passer les matières solides ou liquides.

10. Moyen suivant l'une des revendications 1 à 9, caractérisé en ce que l'épaisseur du revêtement (10) est comprise entre 0,5 et 1,0 millimètre.

11. Moyen suivant l'une des revendications 1 à 10, caractérisé en ce que le revêtement (10) est constitué d'une matière stérilisable.

12. Moyen suivant l'une des revendications 1 à 11, caractérisé en ce que la couche (11) située du côté de l'os enrobe, sur leurs bords (12a), la couche intermédiaire et/ou la couche (12) située du côté de la cavité, au moins dans une zone partielle.

13. Moyen suivant l'une des revendications 1 à 12, caractérisé en ce que la couche (11) située du côté de l'os est constituée de cellulose oxydée régénérée ou de collagène bovin natif, ou thrombine.

14. Moyen suivant l'une des revendications 1 à 13, caractérisé en ce que la couche (12) située du côté de la cavité est constituée de métal ou de matière plastique.

15. Moyen suivant l'une des revendications 1 à 14, caractérisé en ce que la spatule (16) peut être raccordée par conjugaison de formes au revêtement (10).
